# FASCICULE DE BREVET EUROPEEN

(11) **EP 3 009 113 B1**
(45) Date de publication et mention de la délivrance du brevet: **09.06.2021**
(21) Numéro de dépôt: 15002846.2
(22) Date de dépôt: 06.10.2015
(51) Int. Cl.: A61F 9/02

(54) **MASQUE À ÉCRAN AMOVIBLE**
MASKE MIT ABNEHMBAREN BILDSCHIRM
MASK WITH REMOVABLE SCREEN

(30) Priorité: 16.10.2014 FR 1402337
(43) Date de publication de la demande: 20.04.2016
(73) Titulaire: ATOMIC Austria GmbH, 5541 Altenmarkt im Pongau (AT)
(72) Inventeur: Montagnoni, Thibaut, 74330 Lovagny (FR); Lapierre, Christophe, 74000 Annecy (FR)
(74) Mandataire: Lapierre, Stéphane

(56) Documents cités:
- WO-A1-2014/065528
- WO-A1-2014/076377
- DE-U1-202013 000 104
- US-A1- 2005 015 862
- US-A1- 2009 038 059
- US-A1- 2014 033 408

## Description

L'invention concerne les masques de protection oculaire, en particulier les masques de protection pour la pratique de sports en plein air, tels que le ski ou surf alpin, le vélo tout terrain ou le motocross.

Des masques de protection sont habituellement utilisés durant la pratique de certains sports de plein air pour protéger les yeux de l'utilisateur du vent, de l'eau, de la boue ou du rayonnement solaire.

Un tel masque comprend généralement une armature supportant un écran de protection transparent et une sangle fixée de part et d'autre de l'armature. La sangle permet de maintenir le masque sur le visage en entourant partiellement le crâne de l'utilisateur. Des éléments de confort peuvent être ajoutés à l'armature pour améliorer le confort du port du masque.

L'utilisateur peut souhaiter remplacer l'écran pour diverses raisons. Par exemple, il peut vouloir changer l'indice de filtration des rayonnements solaires, en fonction des conditions météorologiques. Il peut aussi remplacer un écran endommagé. Ce peut être aussi pour des raisons esthétiques, l'utilisateur voulant un écran correspondant aux dernières tendances de la mode.

Pour répondre à ce besoin, il existe de nombreuses solutions connues assurant cette interchangeabilité. Beaucoup de ces solutions sont complexes, nécessitent une multitude de pièces et ne sont pas toujours ergonomiques.

Le document US 2009/0222979 décrit un masque comprenant un écran muni de clips venant coopérer avec une armature. Pour retirer l'écran, il faut alors exercer un effort directement sur l'écran ou sur le clip, ou encore déformer l'armature. Ce n'est pas une opération très pratique et facile. De plus, le dimensionnement du clip est généralement petit ce qui ne facilite pas la manipulation.

Le document US 2014/0033408 décrit un masque comprenant une patte fixée à l'écran coopérant avec une lame ressort logée dans une armature de manière à solidariser l'écran avec la lame ressort. Un bouton est également logé dans l'armature et est destiné à solliciter la lame ressort de sorte qu'elle ne puisse plus coopérer avec la patte ce qui permet de retirer l'écran. La patte ne se déforme pas. L'ergonomie est simple mais le dispositif est relativement complexe et encombrant ce qui peut gêner le champ visuel.

Le document WO 2014/065528 décrit un masque comprenant une patte fixée à l'écran coopérant avec un bouton logé dans une armature de manière à solidariser l'écran avec le bouton. Le bouton est sollicité par des ressorts afin de le maintenir dans une configuration dans laquelle il coopère avec la patte. En appuyant sur le bouton, celui-ci ne coopère plus avec la patte ce qui permet de retirer l'écran. La patte ne se déforme pas. L'ergonomie est simple mais le dispositif comprend plusieurs pièces constitutives : patte, bouton et ressorts.

Le dispositif est également encombrant ce qui peut gêner le champ visuel.

Un masque selon le préambule de la revendication 1 est connu des documents WO-A-2014/065528 et DE-U1-20 2013 000104.

Le but de l'invention est de proposer un masque amélioré.

Un but est notamment de proposer un masque dont l'écran est amovible et dont le mécanisme de libération est simple et ergonomique.

Un autre but est de proposer un masque dont le mécanisme de libération de l'écran est fiable.

Un autre but est de proposer un masque constitué d'un nombre réduit de pièces.

Un autre but est de proposer un masque compact offrant un bon champ visuel.

L'invention propose un masque de protection oculaire pour la pratique de sports en plein air comprenant :
- une armature délimitant une ouverture, l'armature supportant au moins un premier moyen d'accroche,
- un écran de protection oculaire destiné à obturer l'ouverture, l'écran supportant au moins un deuxième moyen d'accroche, le deuxième moyen d'accroche étant apte à coopérer directement avec le premier moyen d'accroche de manière à maintenir l'écran solidaire de l'armature selon au moins une direction.

Le masque est caractérisé par le fait qu'il comprend en outre un bouton apte à solliciter un moyen d'accroche de manière à provoquer la libération de l'écran de l'armature, le bouton étant monté sur la pièce ne supportant pas le moyen d'accroché pouvant être sollicité par le bouton.

Ainsi, cette conception d'accroche de l'écran sur l'armature offre une solution simple de solidarisation d'un écran avec une armature qui permet une mise en place et un retrait très facile et rapide de l'écran. Le nombre de pièces est réduit à deux éléments : un clip formant le moyen d'accroche sollicité par un bouton. Le bouton sert à désactiver les moyens d'accroche en agissant sur l'un d'entre eux. Lorsque le bouton est apte à solliciter le premier moyen d'accroche, le bouton est logé sur l'écran. A l'inverse, lorsque le bouton est apte à solliciter le deuxième moyen d'accroche, le bouton est logé sur l'armature. Cette dissociation de support entre le bouton et le moyen d'accroche pouvant être sollicité permet de simplifier le mécanisme et de faciliter l'opération de retrait de l'écran. Cela permet aussi de réduire l'encombrement du mécanisme de désactivation des moyens d'accroche. La cinématique de ce mécanisme est simple.

L'écran coopère directement avec l'armature, sans pièce intermédiaire, ce qui renforce l'accroche et fiabilise le dispositif de maintien de l'écran. De plus, cette conception permet d'utiliser que des pièces plastiques.

Selon des aspects avantageux mais non obligatoires de l'invention, un tel masque peut incorporer une ou plusieurs des caractéristiques suivantes, prises dans toute combinaison techniquement admissible :
- Le moyen d'accroche sollicité par le bouton est le deuxième moyen d'accroche supporté par l'écran.
- Le premier moyen d'accroche comprend un logement et le deuxième moyen d'accroche comprend un clip, une surface d'arrêt, ménagée sur le clip, étant apte à coopérer avec une surface d'arrêt complémentaire, ménagée dans le logement. Les moyens d'accroche comprennent un clip supporté par l'écran et un logement supporté par l'armature, une surface d'arrêt, ménagée sur le clip, étant apte à coopérer avec une surface d'arrêt complémentaire, ménagée dans le logement. Selon un mode de réalisation, le clip est sollicité élastiquement de sorte à pouvoir revenir vers une position stable dans laquelle la surface d'arrêt du clip est apte à coopérer avec la surface d'arrêt complémentaire du logement.
- Une partie de l'armature constitue le premier moyen d'accroche.
- L'armature comprend un logement de guidage du bouton. Selon un mode de réalisation, le bouton comprend au moins une surface de butée apte à coopérer avec au moins une surface de butée complémentaire ménagée sur le logement de guidage du bouton de manière à limiter le déplacement du bouton dans un sens.
- Le bouton et/ou le deuxième moyen d'accroche comprend un moyen d'extraction provoquant l'éloignement d'une partie de l'écran de l'armature, lorsque le bouton est actionné.
- Le moyen d'extraction comprend une pente d'introduction ménagée sur le clip et agencée de sorte que la pente puisse coopérer avec le bouton, lorsque le bouton sollicite le clip, afin de générer un effort sur le clip dans une direction provoquant l'éloignement de l'écran de l'armature au niveau du clip.
- Le moyen d'extraction comprend une pente d'éjection ménagée sur le bouton et agencée de sorte que la pente puisse coopérer avec le clip, lorsque le bouton sollicite le clip, afin de générer un effort sur le clip dans une direction provoquant l'éloignement de l'écran de l'armature au niveau du clip.
- Le bouton se déplace selon une direction sensiblement verticale.
- Un moyen d'accroche est déformable par le bouton lorsqu'on actionne celui-ci. Selon un mode de réalisation, le masque comprend une butée permettant de limiter la déformation d'un moyen d'accroche.
- La tête du bouton est logée dans une cavité du masque.
- Le bouton est positionné au niveau d'une extension latérale de l'armature. Selon un mode de réalisation, le masque comprend au moins quatre boutons aptes à coopérer avec au moins quatre clips, chaque extension latérale de l'armature supportant deux boutons montés en opposition.
- Le bouton est logé dans la partie centrale de l'écran.

D'autres caractéristiques et avantages de l'invention seront mieux compris à l'aide de la description qui va suivre, en regard des dessins annexés illustrant, selon des formes de réalisation non limitatives, comment l'invention peut être réalisée, et dans lequel :

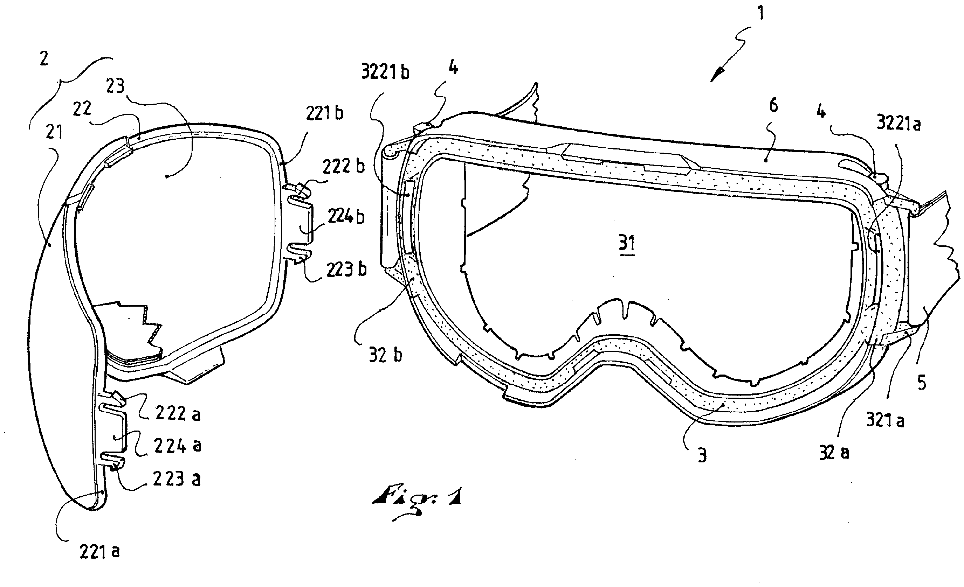

- la figure 1 est une vue en perspective avant éclatée d'un masque selon l'invention ;
- la figure 2 est une vue de face du masque assemblé ;
- la figure 3 est une vue de dessus du masque assemblé ;
- les figures 4 à 7 sont des vues en coupes partielles selon IV-IV de la figure 3 illustrant les différentes étapes d'assemblage de l'écran sur l'armature ;
- la figure 8 est une vue en perspective avant éclatée d'un masque selon un deuxième mode de réalisation de l'invention ;
- la figure 9 est une vue en coupe médiane verticale du masque assemblé selon le deuxième mode de réalisation de l'invention.

Le masque 1 comprend une armature 3. L'armature 3 délimite une ouverture 31. Dans l'exemple, cette ouverture couvre, quand le masque est porté, une zone de vision s'étendant d'une tempe à l'autre, du front aux pommettes. L'ouverture 31 est obturée par un écran de protection oculaire 2. Cet écran est fixé à l'armature par des moyens d'accroche 222, 223, 322. L'écran 2 est assemblé sur l'armature 3 selon une direction horizontale, c'est-à-dire, selon une direction sensiblement perpendiculaire à l'ouverture 31. Une sangle 5 est fixée aux extrémités latérales de l'armature 3. La sangle 5 et l'armature 3 forment une boucle destinée à entourer le crâne de l'utilisateur pour assurer le maintien en place du masque.

Dans la suite de la description, il sera fait usage de termes tels que « horizontal », « vertical », « transversal », « supérieur », « inférieur », « latéral », « haut », « bas », « droite », « gauche », « avant », « arrière », « devant », « derrière ». Ces termes doivent être interprétés en fait de façon relative en relation avec la position normale que le masque occupe sur le visage de l'utilisateur quand il se tient debout, tête droite. Par « arrière », on désigne les parties orientées côté tête / yeux de l'utilisateur. On utilisera l'indice « a », en référence aux éléments de la partie gauche du masque et l'indice « b », en référence aux éléments de la partie droite du masque.

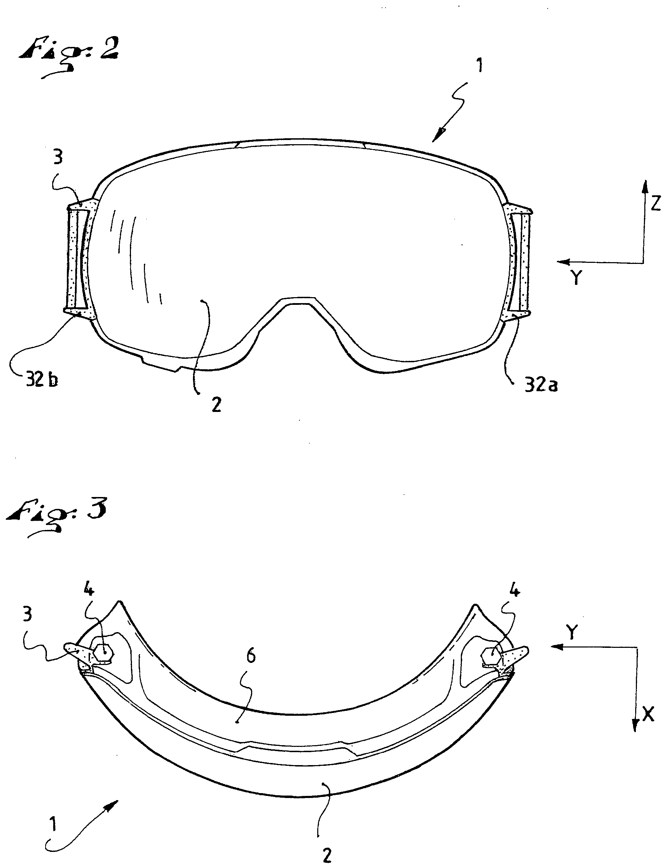

Les figures 2 et 3 illustrent les principales directions. La direction avant/arrière correspond à l'axe X. La direction transversale ou droite/gauche correspond à l'axe Y. La direction verticale ou haut/bas correspond à l'axe Z.

L'écran de protection 2 obture l'ouverture 31 de l'armature 3 afin de protéger les yeux de l'utilisateur d'agressions extérieures telles que le vent, la neige ou l'eau.

Dans cet exemple, les moyens d'accroche 222, 223, 322 permettant de solidariser l'écran 2 sur l'armature 3 comprennent au moins un premier moyen d'accroche 322 supporté par l'armature et au moins un deuxième moyen d'accroche 222, 223 supporté par l'écran. Ici, chaque premier moyen d'accroche est réalisé par un clip solidaire de l'écran et chaque deuxième moyen d'accroche est réalisé par un logement de l'armature. Dans le mode de réalisation, l'écran comprend quatre clips coopérant avec deux logements correspondant de l'armature. Ces premier - 322 - et deuxième moyens d'accroches 222, 223 seront détaillés par la suite. Pour simplifier la description, seule la construction de la partie gauche du masque sera illustrée. Cette construction s'applique mutatis mutandis à la partie droite du masque, symétriquement.

Dans ce mode de réalisation, l'écran de protection 2 comprend un panneau optique externe 21 et un panneau optique interne 23 assemblés à un cadre 22.

Les panneaux optiques sont habituellement réalisés en matière synthétique, par exemple, en polycarbonate ou acrylique. Ils sont transparents et disposés en vis-à-vis des yeux de l'utilisateur.

Le panneau optique externe 21 peut présenter de façon connue en soi des propriétés de filtration lumineuse, afin d'assurer la protection des yeux de l'utilisateur contre le rayonnement solaire. Le panneau optique externe 21 peut par exemple assurer la filtration des ultraviolets.

Le panneau optique interne 23 peut, par exemple, présenter des propriétés pour limiter la condensation de la vapeur d'eau.

Le cadre 22 forme une boucle reproduisant sensiblement le pourtour des panneaux optiques 21, 23. Ce cadre est fixé sur la face intérieure du panneau optique externe 21 et sur la face externe du panneau optique 23, par des moyens appropriés comme par exemple, du collage, une soudure ou une vis. Ainsi, le cadre 22 est disposé entre les deux panneaux optiques 21, 23, il est pris en sandwich entre ces panneaux optiques.

Dans une variante, l'écran de protection comprend un seul panneau optique ou plus de deux panneaux optiques.

Le cadre 22 comprend deux parties latérales 221a, 221b. Pour chaque partie latérale 221a, 221b du cadre, celle-ci comprend un clip supérieur 222a,222b et un clip inférieur 223a, 223b. Ces deux clips 222a, 222b, 223a, 223b forment, dans cet exemple, des deuxièmes moyens d'accroche de l'écran à l'armature.

Pour simplifier la description, nous allons décrire par la suite le mécanisme d'accroche de l'écran comportant le clip supérieur de la partie latérale gauche 222a. Il est entendu que la construction est analogue pour les trois autres clips 222b, 223a, 223b, à l'orientation près.

Au sens de l'invention, un clip 222a forme une patte 2221a faisant saillie d'un composant de l'écran selon une direction d'extension L. Cette patte comprend une extrémité libre formant un crochet 2222a définissant une surface d'arrêt 2223a sensiblement perpendiculaire à la direction d'extension de la patte 2221a quand le clip est dans une position au repos stable, dite configuration en prise. La surface d'arrêt est orientée en direction du cadre 22. Comme nous le verrons par la suite, cette surface d'arrêt est destinée à coopérer avec une surface d'arrêt complémentaire 3222a d'un logement 322a de l'armature 3 afin de maintenir l'écran 2 solidaire de l'armature 3. Lorsque la surface d'arrêt du crochet coopère avec la surface d'arrêt complémentaire, le crochet est dans sa configuration en prise. Par ailleurs, le clip est conçu pour que le crochet soit mobile entre cette configuration en prise et une configuration de libération. Lorsque le crochet est dans sa configuration de libération, il est agencé de manière à ne plus coopérer avec l'armature. Dans ce cas, la surface d'arrêt du crochet est positionnée de manière à ne plus être en vis-à-vis de la surface d'arrêt complémentaire de l'armature. L'écran peut alors être retiré de l'armature. Le clip comprend un moyen élastique permettant de ramener le crochet vers une position stable correspondant à sa configuration en prise.

Dans l'exemple illustré, un clip 222a s'étend en saillie à partir du cadre. Du fait du matériau utilisé, le clip est déformable élastiquement. Comme nous le verrons par la suite, le clip peut être sollicité élastiquement de sorte à pouvoir revenir vers une position stable dans laquelle la surface d'arrêt 2223a du clip est apte à coopérer avec la surface d'arrêt complémentaire 3222a du logement 322a de l'armature 3. Ainsi, l'élasticité du clip assure le retour élastique. Le clip s'étend vers l'arrière de l'écran sensiblement selon la direction X, vers l'extérieur du cadre, dans un plan sensiblement horizontal. Dans le plan horizontal, le clip peut être orienté à plus ou moins 30° par rapport à la direction X. Chaque clip se déforme selon une direction verticale, correspondant à une flexion selon un axe sensiblement transversal, correspondant à une direction Y. Le clip supérieur 222a se déforme vers le bas et le clip inférieur 223a se déforme vers le haut. En conséquence, le clip supérieur est construit en opposition au clip inférieur par rapport à un plan médian horizontal au cadre. Le clip supérieur 222a est fixé sur la moitié supérieure de la partie latérale correspondante 221a et le clip inférieur 223a est fixé sur la moitié inférieure de la partie latérale correspondante 221a.

Un clip est prévu pour fléchir lorsqu'on exerce un effort sur une face d'actionnement, en l'occurrence, au niveau du crochet. On désigne par « faces externes » d'un clip, les faces parallèles de la patte et du crochet orientées du côté de la face d'actionnement. Par opposition, on désigne par « faces internes » d'un clip les faces parallèles de la patte et du crochet opposées aux faces externes. Ainsi, un clip se déforme en direction de ses faces internes. Dans cet exemple, les faces externes sont les faces horizontales parallèles les plus éloignées du plan médian horizontal au cadre. Cela correspond aux faces supérieures pour les clips supérieurs et aux faces inférieures pour les clips inférieurs. A l'inverse, les « faces internes » correspondent aux faces horizontales parallèles les plus proches du plan médian horizontal au cadre.

Dans cet exemple, le crochet 2222a intègre une pente d'introduction 2224a entre la face sommitale du clip et la face externe du crochet. L'angle de la pente d'introduction par rapport à un plan horizontal est compris entre 15 et 60°, et de préférence 30°. Ainsi, un effort horizontal sur la pente d'introduction génère une composante verticale en réaction, tendant à faire fléchir le clip correspondant.

Selon un mode de réalisation, le cadre 22 comprend, sur chaque partie latérale 221a, 221b, une saillie de butée 224a, 224b. Chaque saillie de butée est disposée dans la partie médiane de la partie latérale correspondante, entre le clip supérieur et le clip inférieur. Cette saillie de butée est dimensionnée de sorte qu'après une certaine déformation du clip, une partie du clip vient en contact avec la saillie de butée empêchant ainsi le clip de se déformer davantage. Cette limitation de la déformation du clip permet de ne pas l'endommager. Le clip conserve ainsi toujours une course élastique et ne subit pas de déformation plastique. De plus, cela permet d'éviter de casser le clip par une manipulation involontaire de l'utilisateur, lorsque l'écran n'est pas assemblé sur l'armature. Cette saillie de butée peut également servir pour la préhension de l'écran, ce qui permet d'éviter un contact salissant entre les doigts et les panneaux optiques.

Dans une variante non illustrée, les clips peuvent être des pattes articulées rapportées sur le cadre. Dans ce cas, avantageusement, un moyen élastique permet de configurer les clips dans une configuration en prise.

Alternativement, les clips forment une extension d'un panneau optique.

Selon une autre variante, les clips sont sur un ou plusieurs supports, différent(s) du cadre, rapporté(s) sur un panneau optique.

Le masque comprend par ailleurs, une armature 3 formant un encadrement délimitant une ouverture 31 destinée à être obturée par l'écran 2 lorsque le masque est assemblé. L'armature est généralement constituée d'un matériau rigide.

Dans ce mode de réalisation, l'armature comprend deux extensions latérales 32a, 32b. Nous allons décrire l'extension latérale 32a de la partie gauche de l'armature, étant entendu que l'autre extension latérale 32b est construite symétriquement de manière analogue. L'extension latérale 32a supportant chacune une boucle d'attache 321a de la sangle 5. Chaque extension latérale, comprend un logement 322a pour recevoir une paire de clips latéraux 222a, 223a. Le logement 322a comprend un orifice 3221a traversant l'extension latérale selon une direction X. La hauteur minimale h de l'orifice est sensiblement supérieure à la distance d1 entre les faces externes des pattes des clips latéraux 222a, 223a, et est sensiblement inférieure à la distance maximale d2 entre les faces externes des crochets des clips latéraux 222a, 223a, lorsque le clip n'est pas sollicité. Le logement 322a comprend également une surface d'arrêt complémentaire 3222a sensiblement perpendiculaire à la direction d'insertion X des clips correspondant à l'axe de l'orifice 3221a. Cette surface d'arrêt complémentaire 3222a s'étend verticalement, de part et d'autre de l'orifice, côté extérieur, c'est-à-dire, côté opposé à l'ouverture 31 de l'armature 3. Ce logement 322a forme, dans cet exemple, un premier moyen d'accroche de l'écran à l'armature.

Avantageusement, chaque logement 322a comprend également deux pentes d'insertion 3223a disposées en opposition, chacune s'étendant, de part et d'autre de l'orifice, côté intérieur, c'est-à-dire, du côté de l'ouverture 31 de l'armature 3. L'angle d'une pente d'insertion par rapport à un plan horizontal est compris entre 15 et 60°, et de préférence 30°, de sorte à réduire la hauteur de l'orifice en direction de l'arrière. Ainsi, lorsque le crochet du clip vient en contact avec cette pente lors d'un mouvement de translation selon une direction horizontale X, ce déplacement entraîne, en réaction, un déplacement vertical de l'extrémité du clip tendant à faire fléchir le clip correspondant.

Dans cet exemple, chaque extension latérale 32a comprend deux logements de guidage 323a, un logement de guidage supérieur et un logement de guidage inférieur, s'étendant verticalement, de part et d'autre de l'orifice, côté extérieur, c'est-à-dire, côté opposé à l'ouverture 31 de l'armature 3. Les deux logements de guidage d'une extension latérale sont symétriques par rapport à un plan médian horizontal XY de l'armature. En conséquence, on va décrire un seul logement de guidage, par exemple, le logement de guidage supérieur, le deuxième logement de guidage étant de construction symétrique par rapport au plan médian horizontal XY.

Dans l'exemple, le logement de guidage supérieur reproduit un profilé en forme de « U », s'étendant sensiblement verticalement délimité par trois parois : une paroi avant, une paroi arrière et une paroi latérale positionnée du côté de l'ouverture 31. En conséquence, la surface d'arrêt complémentaire 3222a forme une partie de la surface interne de la paroi latérale du logement de guidage. L'extrémité supérieure du logement de guidage comprend un rebord 3231a s'étendant horizontalement vers l'intérieur du logement de guidage créant ainsi un rétrécissement de section à ce niveau. Le rebord 3231a forme ainsi un orifice de passage 3232a ayant une section inférieure à la section interne principale du logement de guidage. Le rebord 3231a comprend une face inférieure formant une surface de butée complémentaire interne 3236a et une face supérieure formant une surface de butée complémentaire externe 3235a. Le logement de guidage est destiné à recevoir un bouton 4 permettant d'agir sur les moyens d'accroché 222, 223 du masque.

Le masque 1 comprend ainsi au moins un bouton 4, dans cet exemple, quatre, permettant de libérer l'écran de l'armature. Le bouton comprend une tête 41 reliée à un corps 42 par une zone de liaison 43. La zone de liaison s'étend sur une hauteur h1 et présente une section inférieure aux sections du corps et de la tête. La hauteur h1 de la zone de liaison 43 est supérieure à l'épaisseur e du rebord 3231a. La section de la zone de liaison 43 est sensiblement inférieure à la section de l'orifice de passage 3232a du logement de guidage. La section de la zone de liaison 43 est dimensionnée pour se loger, sans interférer, dans la section de l'orifice de passage 3232a. La section du corps 42 est sensiblement supérieure à la section de l'orifice de passage 3232a du logement de guidage et sensiblement inférieure à la section interne principale du logement de guidage. La section du corps 42 est dimensionnée pour se loger, sans interférer, dans la section interne principale du logement de guidage. La section de la tête 41 est sensiblement supérieure à la section de l'orifice de passage 3232a du logement de guidage. La tête 41 comprend une surface de butée externe 45, orientée vers le corps, correspondant à la face horizontale inférieure de la tête. A l'opposé de la surface de butée externe, la tête comprend une surface d'appui pour un doigt, correspondant à la face horizontale supérieure de la tête. Cette surface est dimensionnée pour être ergonomique au toucher afin de faciliter l'actionnement du bouton. Par exemple, la surface est plane ou légèrement bombée en creux et sa taille est supérieure à 25 mm² afin que l'actionnement du bouton ne nécessite pas une forte pression de contact, source d'inconfort. Le corps 42 comprend une surface de butée interne 46, orientée vers la tête, correspondant à la face horizontale supérieure du corps. Les surfaces de butée interne - 46 - et externe 45 se font face et délimitent la zone de liaison 43.

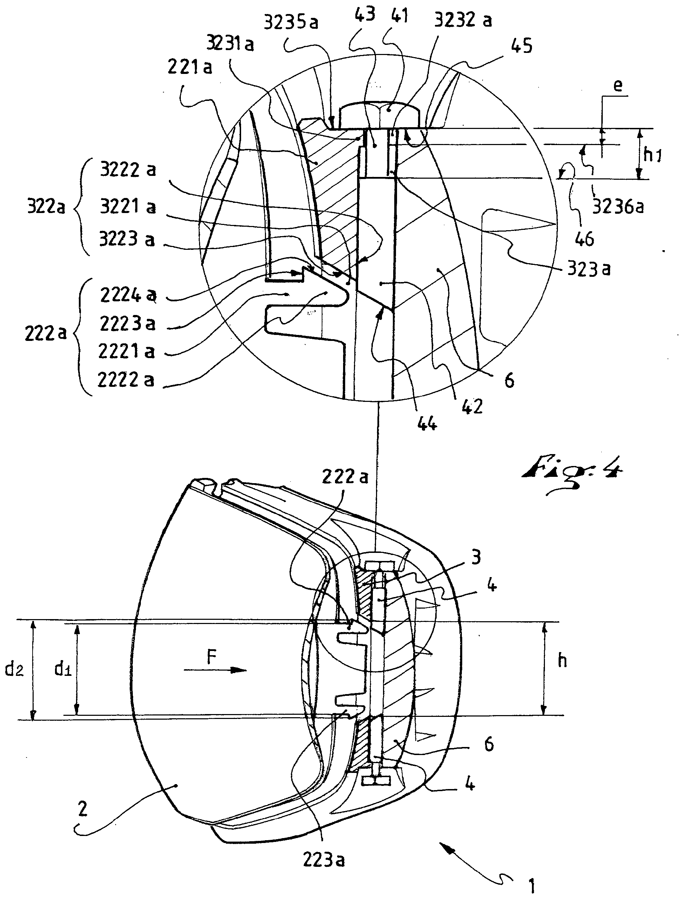

Lorsque le bouton 4 est assemblé dans l'armature 3, le corps 42 est placé dans le logement de guidage 323a, la zone de liaison 43 est placée dans l'orifice de passage 3232a et la tête 41 dépasse de l'extension latérale 32. Le bouton 4 peut se déplacer selon une direction sensiblement verticale. La course de déplacement du bouton est cependant limitée par le rebord 3231a comme on le voit dans les figures 4 à 7. Dans un sens, le déplacement est limité lorsque la surface de butée externe 45 du bouton 4 vient en contact avec la surface de butée complémentaire externe 3235a du rebord 3231a. Dans l'autre sens, le déplacement est limité lorsque la surface de butée interne 46 du bouton 4 vient en contact avec la surface de butée complémentaire interne 3236a du rebord 3231a. La course de déplacement du bouton correspond alors à la hauteur h1 de la zone de liaison 43 moins l'épaisseur e du rebord 3231a.

Le bouton 4 est prévu pour solliciter en flexion un clip associé 222a lors d'une action manuelle P de l'utilisateur. L'extrémité libre du corps 42, c'est-à-dire l'extrémité la plus éloignée de la tête 41, est alors apte à venir en contact avec le crochet 2222a du clip 222a. En conséquence, le bouton 4 doit permettre, d'une part, la coopération entre les moyens d'accroche, quand le bouton n'est pas actionné, et d'autre part, faire fléchir le clip pour qu'il ne coopère plus avec le logement 322 de l'armature 3, quand le bouton est actionné. Le positionnement du clip quand l'écran est assemblé à l'armature, la course de déplacement du bouton ainsi que son positionnement sont dimensionnés pour assurer ces deux fonctions.

Selon un mode de réalisation, le positionnement du clip quand l'écran est assemblé à l'armature, la course de déplacement du bouton ainsi que son positionnement sont dimensionnés de sorte que le clip est légèrement contraint par le bouton ou en contact direct avec celui-ci, en configuration assemblé. Cet agencement permet de limiter le déplacement du bouton dans son logement de guidage lorsque le masque est assemblé. Le bouton ne ballote pas ou très peu. La qualité perçue est meilleure.

Alternativement, le masque comprend un moyen élastique, par exemple un ressort, permettant de pousser le bouton de sorte que la surface de butée interne appuie contre la surface de butée complémentaire interne lorsque le bouton n'est pas sollicité par l'utilisateur.

Selon un mode de réalisation, le bouton 4 comprend une pente d'éjection 44 définissant une surface en biais au niveau de l'extrémité libre du corps 42. L'angle d'inclinaison de la pente d'éjection par rapport à un plan horizontal est compris entre 15 et 60°, et de préférence 30°. Par ailleurs, le bouton 4 est indexé angulairement par rapport à son axe longitudinal de sorte que l'angle d'inclinaison est défini par rapport à un axe sensiblement transversal Y de l'armature et que la pente soit orientée vers l'avant du masque. L'indexation est réalisée, par exemple, par une section non circulaire du corps du bouton logée dans une section complémentaire du logement de guidage. Ces sections sont, par exemple, polygonales. La pente 44 est dimensionnée pour venir en contact avec le crochet 2222a du clip 222a. Ainsi, lorsque l'utilisateur appuie sur le bouton 4 selon une direction verticale Z, la pente entre en contact avec l'extrémité du clip. Le déplacement du bouton entraîne alors, en réaction, un effort sur le clip en direction de l'avant du masque. Cet effort permet de faciliter l'éjection de l'écran par rapport à l'armature dès que les moyens d'accroche ne coopèrent plus entre eux. Le clip, et donc l'écran, est ainsi sollicité vers l'avant ce qui permet d'éloigner l'écran de l'armature. L'écran peut facilement être retiré. On notera que la pente d'introduction 2224a d'un clip produit le même effet, favorisant ainsi l'éjection de l'écran de l'armature. Dans l'exemple illustré, la pente d'introduction 2224a d'un clip coopère avec la pente d'éjection 44 du bouton associé. Cette coopération de deux pentes amplifie le transfert de mouvement d'une pièce à l'autre selon une direction sensiblement perpendiculaire.

L'éjection de l'écran est également accentuée du fait de l'élasticité du clip. En effet, lorsque le clip revient vers sa position d'équilibre, il exerce une force verticale à son extrémité. Lorsque cette extrémité est en contact avec une surface inclinée, l'effort de rappel du clip se transpose en effort horizontal d'éjection. Tant que le clip est maintenu horizontalement par les moyens d'accroche, le clip emmagasine l'effort de rappel et ne le restitue pas en effort d'éjection. Dès que les moyens d'accroche sont désactivés, l'effort de rappel est transformé en effort d'éjection comme expliqué précédemment.

Il convient de noter que la pente d'insertion 3223a du logement 322a de l'armature contribue également à l'éjection du masque. Le principe de transfert d'effort du clip avec cette pente d'insertion est analogue au transfert d'effort du clip avec la pente d'éjection du bouton. La pente d'insertion peut donc être aussi considérée comme un élément favorisant l'éloignement d'une partie de l'écran de l'armature.

Cette construction présente ainsi l'avantage de faciliter le retrait de l'écran en intégrant des moyens d'extraction simple permettant d'écarter l'écran de l'armature. Les moyens d'extraction sont ici réalisés par la pente d'introduction 2224 d'un clip 222, 223, la pente d'éjection 44 d'un bouton 4, la pente d'insertion 3223 du logement 322 et l'élasticité du clip. Pour faciliter l'éloignement d'une partie de l'écran de l'armature, il n'est pas nécessaire de cumuler tous ces moyens d'extraction, au moins un peut suffire.

Dans cet exemple, une jupe 6 est fixée sur l'armature 3. Cette jupe est accrochée sur tout le pourtour de l'armature 3 et s'étend en direction du visage de l'utilisateur. La jupe 6 est généralement en matière souple. Avantageusement, la jupe 6 comprend, de chaque côté, une surface latérale sensiblement verticale agencée de manière à fermer le logement de guidage 323a quand la jupe 6 est assemblée à l'armature 3. Ainsi, dans cette configuration assemblée, le bouton 4 reste continuellement solidaire du masque 1.

Les figures 4 à 7 illustrent le mécanisme d'assemblage de l'écran sur l'armature et notamment les moyens d'accroche 222, 223, 322.

Dans un premier temps, on approche l'écran en direction de l'armature comme représenté par la flèche F dans la figure 4. Dans cette configuration, de par la gravité, le bouton supérieur 4 est maintenu solidaire de l'armature grâce à la coopération entre la surface de butée externe 45 du bouton 4 et la surface de butée complémentaire externe 3235a du rebord 3231a et le bouton inférieur 4 est maintenu solidaire de l'armature grâce à la coopération entre la surface de butée interne 46 du bouton 4 et la surface de butée complémentaire interne 3236a du rebord 3231a. La tête 41 du bouton supérieur 4 appuie sur l'extension latérale 32a de l'armature. La pente d'éjection 44 du bouton supérieur 4 sort du logement de guidage 323a pour se positionner en vis-à-vis de l'orifice traversant 3221a du logement 322a de l'armature, en alignement avec la pente d'introduction 2224a du clip supérieur 222a. La tête 41 du bouton inférieur 4 est écartée de l'extension latérale 32a de l'armature. La pente d'éjection 44 du bouton inférieur 4 reste à l'intérieur du logement de guidage 323a.

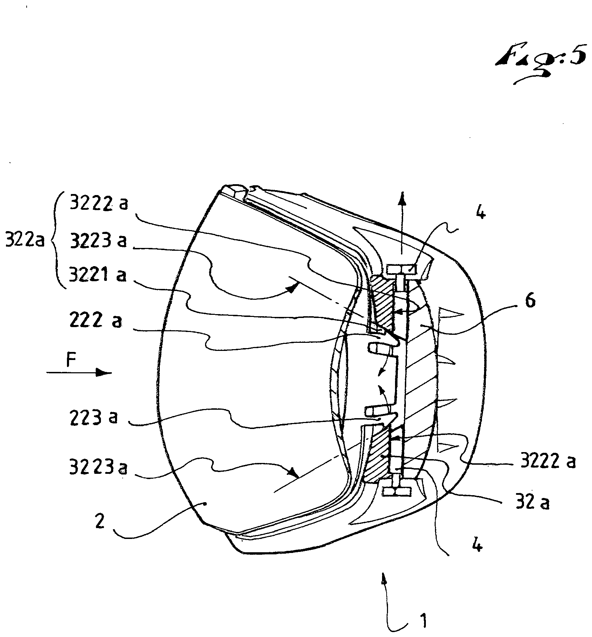

Le clip supérieur 222a et le clip inférieur 223a viennent alors en contact avec l'armature 3. Plus précisément, les pentes d'introductions 2224a des crochets 2222a des clips 222a, 223a s'appuient sur les pentes d'insertion 3223a correspondantes du logement 322a de l'extension latérale 32a de l'armature 3. En poursuivant le rapprochement relatif entre l'écran et l'armature, la coopération des pentes entraîne le fléchissement des clips 222a, 223a. Cette configuration est illustrée à la figure 5.

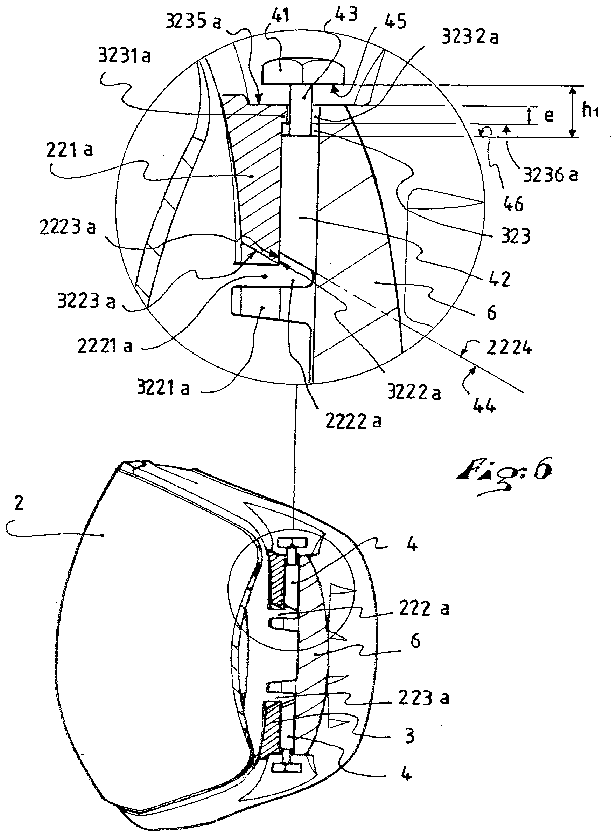

Dès que les clips 222a, 223a atteignent une position d'enclenchement en relation avec le logement 322a, les pentes 2224a et 3223a ne coopèrent plus entre elles. Les clips reviennent vers une position stable correspondant à une configuration en prise comme on le voit à la figure 6. Dans cette configuration, les surfaces d'arrêt 2223a des clips 222a, 223a sont positionnées en vis-à-vis des surfaces d'arrêt complémentaire 3222a du logement 322a. Elles peuvent donc coopérer entre elles pour maintenir les clips dans le logement. Ainsi, l'écran est maintenu solidaire de l'armature selon une direction X. A noter que l'enclenchement des clips provoque également le déplacement du bouton supérieur 4 du fait du contact entre la pente d'introduction 2224a du clip 222a et la pente d'éjection 44 du bouton 4. Le bouton inférieur 4 ne se déplace pas car il reste en position basse par gravité.

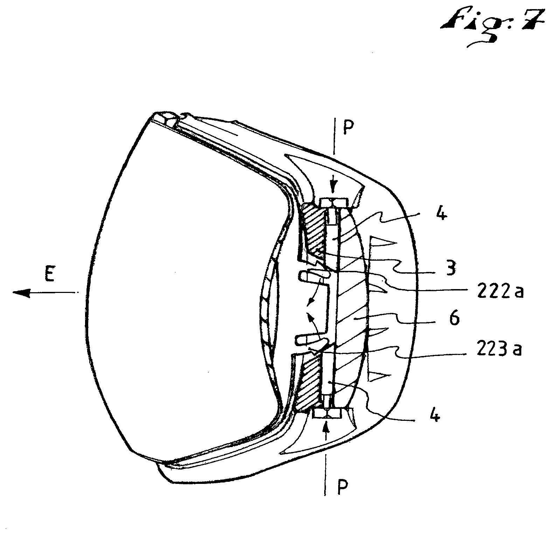

Pour retirer l'écran, il suffit alors d'appuyer simultanément sur la tête 41 des deux boutons 4 comme illustré à la figure 7 par les flèches P. Cette action provoque le déplacement des boutons vers les clips. Une fois en contact, en continuant d'appuyer sur les boutons on entraîne la déformation des clips jusqu'à ce que les surfaces d'arrêt 2223a des clips 222a, 223a ne coopèrent plus avec les surfaces d'arrêt complémentaire 3222a du logement 322a. A ce moment de libération, les clips sont projetés vers l'avant, selon la direction représentée par la flèche E, du fait des pentes d'introduction 2224a en contact avec les pentes d'éjection 45 et de l'élasticité des clips. L'écran 2 est alors désolidarisé de l'armature 3 et même écarté de celle-ci. On peut facilement retirer l'écran.

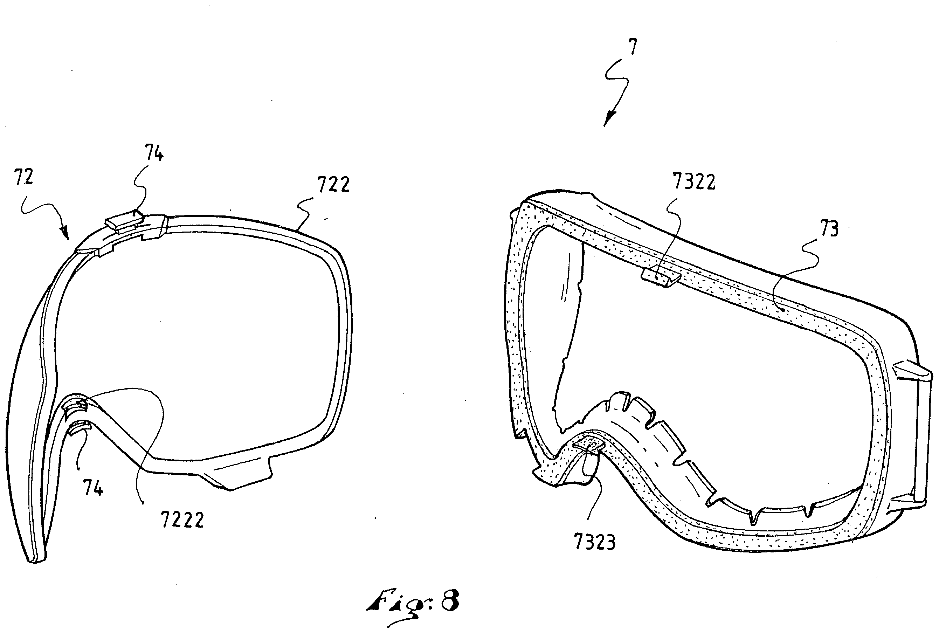

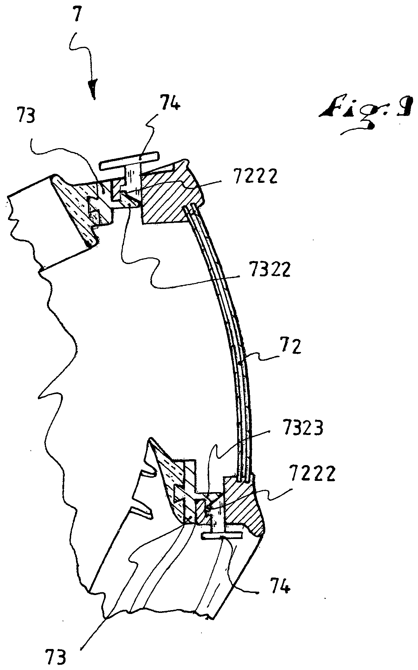

Selon un deuxième mode de réalisation, illustré aux figures 8 et 9, les moyens d'accroche 7322, 7323, 7222 et les boutons associés 74 sont localisés sur la partie centrale de la partie supérieure du masque 7 et sur la partie centrale de la partie inférieure du masque. Dans cet exemple, la construction du masque 7 est analogue mais inversée. Ainsi, l'écran 72 supporte un logement supérieur 7222a et un logement inférieur 7222b. Ces logements 7222 sont supportés par un cadre 722 de l'écran 72 et sont conçus de manière analogue aux logements 322 du premier mode de réalisation. Ces logements 7222 forment les deuxièmes moyens d'accroche. Par ailleurs, l'armature 73 supporte un clip supérieur 7322 et un clip inférieur 7323. Les clips 7322, 7323 sont conçus de manière analogue aux clips 222, 223 du premier mode de réalisation. Ces clips 7222 7322, 7323 forment les premiers moyens d'accroche. Les deux boutons 74 assurant la désactivation des moyens d'accroché en agissant sur les premiers moyens d'accroche sont logés sur l'écran 72. Ainsi, pour retirer l'écran, l'utilisateur appuie sur les deux boutons centraux de l'écran, avec son pouce et son index. Cette action provoque la déformation des clips de l'armature ce qui va permettre la désactivation des moyens d'accroche de l'écran. L'écran peut alors être retiré. Cette construction présente l'avantage de pouvoir maintenir facilement l'écran au moment de son retrait. L'utilisateur a une bonne préhension centrale, sans contact avec les panneaux optiques. Cela permet d'éviter le risque de salissure de l'écran avec ses doigts. L'action de retrait est ergonomique du fait que le geste de rapprochement de ces doigts est naturel. La prise en main est stable.

Dans ces exemples, le premier moyen d'accroche est fixé sur l'armature comme le deuxième moyen d'accroche est fixé sur l'écran. Le premier moyen d'accroche et l'armature forment une pièce unitaire, pouvant être monobloc. Le deuxième moyen d'accroche et l'écran forment également une pièce unitaire, pouvant être monobloc. Il n'y a pas de mouvement relatif possible entre chaque moyen d'accroche et son support autre qu'une déformation du moyen d'accroche. Cela permet de diminuer le nombre de pièces constitutives. La construction est plus compacte et plus robuste.

Dans les modes de réalisation décrits, les boutons se déplacent en translation, dans leur logement de guidage, selon une direction verticale Z. Alternativement, les boutons peuvent se déplacer autrement, selon une direction non verticale, par exemple, légèrement inclinée, ou par une rotation, voire une combinaison de mouvement.

Dans la description, il est fait référence à des directions « sensiblement » verticale, horizontale ou latérale. Par « sensiblement », on englobe des directions variant de plus ou moins 30° par rapport à la direction de référence.

De même, les deux boutons latéraux ne se déplacent pas nécessairement selon une même direction. Les deux boutons ne sont donc pas forcément alignés.

Dans le premier mode de réalisation décrit, chaque extension latérale 32a, 32b de l'armature 3 comprend deux boutons 4 montés en opposition. Cette disposition permet une bonne manipulation ergonomique de l'utilisateur lorsqu'il veut retirer son écran. Cela lui permet d'utiliser son pouce et son index pour appuyer sur les boutons, ce qui est une opération facile à réaliser et présentant une bonne proprioception. Le rapprochement de ces doigts est un geste naturel. L'utilisateur dispose d'une bonne puissance sans grand effort. Par ailleurs, en intégrant les boutons au niveau des extensions latérales, cela permet une meilleure intégration du système dans l'armature et permet d'améliorer l'aspect visuel extérieur du masque, les boutons peuvent être escamotées. Enfin, avec deux boutons latéraux, on augmente le nombre de moyens d'accroche ce qui améliore l'accroche de l'écran sur l'armature.

Dans une variante, chaque extension latérale 32a, 32b comprend un seul bouton agissant sur un ou plusieurs moyens d'accroche. Dans ce cas, il n'y a qu'un seul bouton à actionner. Cela peut simplifier l'opération consistant à retire l'écran.

Dans une autre variante, l'écran est monté pivotant autour d'une charnière localisée au niveau d'une extension latérale et comprend des moyens d'accroche similaires à ceux décrits précédemment au niveau de l'autre extension latérale. Dans ce cas, il peut y avoir un ou deux boutons.

Dans un autre mode de réalisation, un ou plusieurs boutons sont localisés sur la partie supérieure de l'armature et/ou sur la partie inférieure de l'armature.

Avantageusement, la tête du bouton actionnant un moyen d'accroche est logée dans une cavité du masque formée par la jupe et/ou l'armature. Cette cavité est dimensionnée pour être assez large afin de faciliter l'actionnement du bouton par l'utilisateur. Cependant elle est assez haute pour pouvoir entourer au moins partiellement le bouton quand l'écran est assemblé à l'armature. Cela permet d'éviter d'actionner le bouton involontairement et donc d'éjecter l'écran de l'armature. Par exemple, si on fait tomber le masque au niveau des boutons, le contact avec le sol se fera avec les bords de la cavité et non directement avec la tête du bouton.

Dans les modes de réalisation précédents, le maintien est assuré par des clips déformables. On peut cependant envisager d'autres types de moyens d'accroche. Ce peut être, par exemple, un aimant actionnant un bouton, ou une pièce mobile non énergisée.

Comme nous l'avons vu, le bouton peut agir sur le premier moyen d'accroche supporté par l'armature ou sur le deuxième moyen d'accroche supporté par l'écran. Ainsi, le bouton est logé, soit sur l'écran, dans le premier cas, comme illustré dans le deuxième mode de réalisation, soit sur l'armature, dans le deuxième cas, comme illustré dans le premier mode de réalisation.

Toutes ces constructions présentent l'avantage d'être simples et de nécessiter peu de pièces constitutives. Il n'y a besoin d'aucun ressort car on utilise l'énergie du clip pour agir sur les boutons.

L'invention n'est pas limitée à ces modes de réalisation. Il est possible de combiner ces modes de réalisation.

L'invention s'étend également à tous les modes de réalisation couverts par les revendications annexées.

### REFERENCES

1. Masque
2. Ecran de protection
   21. Panneau optique externe
   22. Cadre
      221. Parties latérales
      222. Clips supérieurs
         2221. Pattes
         2222. Crochets
         2223. Surfaces d'arrêt
         2224. Pente d'introduction
      223. Clips inférieurs
      224. Saillie de butée
   23. Panneau optique interne
3. Armature
   31. Ouverture
   32. Extension latérale
      321. Boucle d'attache
      322. Logement
         3221. Orifice traversant
         3222. Surface d'arrêt complémentaire
         3223. Pente d'insertion
      323. Logement de guidage
         3231. Rebord
         3232. Orifice de passage
         3235. Surface de butée complémentaire externe
         3236. Surface de butée complémentaire interne
4. Bouton
   41. Tête
   42. Corps
   43. Zone de liaison
   44. Pente d'éjection
   45. Surface de butée externe
   46. Surface de butée interne
5. Sangle
6. Jupe
7. Masque selon un deuxième mode de réalisation
   72. Ecran
      722. Cadre
      7222. Logement
   73. Armature
      7322. Clip supérieur
      7323 Clip inférieur
   74. Bouton

## Revendications

1. Masque de protection oculaire (1 ; 7) pour la pratique de sports en plein air comprenant :
- une armature (3 ; 73) délimitant une ouverture (31), l'armature supportant au moins un premier moyen d'accroche (322 ; 7322, 7323),
- un écran de protection oculaire (2 ; 72) destiné à obturer l'ouverture, l'écran supportant au moins un deuxième moyen d'accroche (222, 223 ; 7222), le deuxième moyen d'accroche étant apte à coopérer directement avec le premier moyen d'accroche de manière à maintenir l'écran solidaire de l'armature selon au moins une direction (X)
- le masque est pourvu d'un bouton (4 ; 74) apte à solliciter un moyen d'accroche (222/223, 322 ; 7322/7323, 7222) de sorte qu'il ne coopère plus avec l'autre moyen d'accroche (322, 222/223 ; 7222, 7322/7323), **caractérisé en ce que** le bouton est monté sur la pièce (2, 3 ; 72, 73) ne supportant pas le moyen d'accroche pouvant être sollicité par le bouton.

2. Masque de protection oculaire (1) selon la revendication 1, **caractérisé en ce que** le moyen d'accroche sollicité par le bouton est le deuxième moyen d'accroche (222, 223) supporté par l'écran (2).

3. Masque de protection oculaire (1) selon la revendication précédente, **caractérisé en ce que** le premier moyen d'accroche comprend un logement (322) et le deuxième moyen d'accroche comprend un clip (222, 223), une surface d'arrêt (2223), ménagée sur le clip, étant apte à coopérer avec une surface d'arrêt complémentaire (3222), ménagée dans le logement, le clip (222, 223) étant sollicité élastiquement de sorte à pouvoir revenir vers une position stable dans laquelle la surface d'arrêt du clip est apte à coopérer avec la surface d'arrêt complémentaire du logement.

4. Masque de protection oculaire (1) selon l'une des revendications précédentes, **caractérisé en ce qu'**une partie de l'armature constitue le premier moyen d'accroche.

5. Masque de protection oculaire (1) selon l'une des revendications précédentes, **caractérisé en ce que** l'armature comprend un logement de guidage (323) du bouton.

6. Masque de protection oculaire (1) selon la revendication précédente, **caractérisé en ce que** le bouton (4) comprend au moins une surface de butée (45, 46) apte à coopérer avec au moins une surface de butée complémentaire (3235, 3236) ménagée sur le logement de guidage du bouton de manière à limiter le déplacement du bouton dans un sens (Z).

7. Masque de protection oculaire (1, 7) selon l'une des revendications précédentes, **caractérisé en ce que** le bouton et/ou un moyen d'accroche comprend un moyen d'extraction (44, 2224, 3223) provoquant l'éloignement d'une partie de l'écran de l'armature, lorsque le bouton est actionné.

8. Masque de protection oculaire (1, 7) selon la revendication précédente, **caractérisé en ce que** le moyen d'extraction comprend une pente d'introduction (2224) ménagée sur le clip (222, 223 ; 7322, 7323) et agencée de sorte que la pente d'introduction puisse coopérer avec le bouton, lorsque le bouton sollicite le clip, afin de générer un effort sur le clip dans une direction (X) provoquant l'éloignement d'une partie de l'écran de l'armature au niveau du clip.

9. Masque de protection oculaire (1, 7) selon l'une des revendications 6 ou 7, **caractérisé en ce que** le moyen d'extraction comprend une pente d'éjection (44) ménagée sur le bouton (4, 74) et agencée de sorte que la pente d'éjection puisse coopérer avec le clip, lorsque le bouton sollicite le clip, afin de générer un effort sur le clip dans une direction (X) provoquant l'éloignement d'une partie de l'écran de l'armature au niveau du clip.

10. Masque de protection oculaire (1 ; 7) selon l'une des revendications précédentes, **caractérisé en ce qu'**un moyen d'accroche (222, 223 ; 7322, 7323) est déformable par le bouton lorsqu'on actionne celui-ci.

11. Masque de protection oculaire (1) selon la revendication précédente, **caractérisé en ce qu'**il comprend une butée (224a, 224b) permettant de limiter la déformation d'un moyen d'accroche (222, 223)

12. Masque de protection oculaire (1 ; 7) selon l'une des revendications précédentes, **caractérisé en ce que** la tête (41) du bouton est logée dans une cavité du masque.

13. Masque de protection oculaire (1) selon l'une des revendications précédentes, **caractérisé en ce que** le bouton est positionné au niveau d'une extension latérale (32) de l'armature.

14. Masque de protection oculaire (1) selon la revendication précédente, **caractérisé en ce qu'**il comprend au moins quatre boutons (4) aptes à coopérer avec au moins quatre clips (222a, 222b, 223a, 223b), chaque extension latérale (32a, 32b) de l'armature supportant deux boutons montés en opposition.

15. Masque de protection oculaire (7) selon l'une des revendications 1, 7 à 10 ou 12 **caractérisé en ce que** le bouton (74) est logé dans la partie centrale de l'écran (72).

## Patentansprüche

1. Augenschutzmaske (1; 7) für die sportliche Betätigung im Freien, die enthält:
- einen eine Öffnung (31) begrenzenden Rahmen (3; 73), wobei der Rahmen mindestens eine erste Einhakeinrichtung (322; 7322, 7323) trägt,
- einen Augenschutzschirm (2; 72), der dazu bestimmt ist, die Öffnung zu verschließen, wobei der Schirm mindestens eine zweite Einhakeinrichtung (222, 223; 7222) trägt, wobei die zweite Einhakeinrichtung direkt mit der ersten Einhakeinrichtung zusammenwirken kann, um den Schirm gemäß mindestens einer Richtung (X) fest mit dem Rahmen verbunden zu halten,
- die Maske ist mit einem Knopf (4; 74) versehen, der eine Einhakeinrichtung (222/223, 322; 7322/7323, 7222) so beanspruchen kann, dass sie nicht mehr mit der anderen Einhakeinrichtung (322, 222/223; 7222, 7322/7323) zusammenwirkt,
**dadurch gekennzeichnet, dass** der Knopf auf das Bauteil (2, 3; 72, 73) montiert wird, das nicht die Einhakeinrichtung trägt, die vom Knopf beansprucht werden kann.

2. Augenschutzmaske (1) nach Anspruch 1, **dadurch gekennzeichnet, dass** die vom Knopf beanspruchte Einhakeinrichtung die vom Schirm (2) getragene zweite Einhakeinrichtung (222, 223) ist.

3. Augenschutzmaske (1) nach dem vorhergehenden Anspruch, **dadurch gekennzeichnet, dass** die erste Einhakeinrichtung eine Aufnahme (322) enthält und die zweite Einhakeinrichtung einen Clip (222, 223) enthält, wobei eine auf dem Clip eingerichtete Arretierfläche (2223) mit einer in der Aufnahme eingerichteten komplementären Arretierfläche (3222) zusammenwirken kann, wobei der Clip (222, 223) elastisch beansprucht wird, um in eine stabile Stellung zurückkommen zu können, in der die Arretierfläche des Clips mit der komplementären Arretierfläche der Aufnahme zusammenwirken kann.

4. Augenschutzmaske (1) nach einem der vorhergehenden Ansprüche, **dadurch gekennzeichnet, dass** ein Teil des Rahmens die erste Einhakeinrichtung bildet.

5. Augenschutzmaske (1) nach einem der vorhergehenden Ansprüche, **dadurch gekennzeichnet, dass** der Rahmen eine Führungsaufnahme (323) des Knopfes enthält.

6. Augenschutzmaske (1) nach dem vorhergehenden Anspruch, **dadurch gekennzeichnet, dass** der Knopf (4) mindestens eine Anschlagfläche (45, 46) enthält, die mit mindestens einer komplementären Anschlagfläche (3235, 3236) zusammenwirken kann, die auf der Führungsaufnahme des Knopfes eingerichtet ist, um die Verschiebung des Knopfes in einer Richtung (Z) zu begrenzen.

7. Augenschutzmaske (1, 7) nach einem der vorhergehenden Ansprüche, **dadurch gekennzeichnet, dass** der Knopf und/oder eine Einhakeinrichtung eine Entnahmeeinrichtung (44, 2224, 3223) enthält, die das Entfernen eines Teils des Schirms vom Rahmen bewirkt, wenn der Knopf betätigt wird.

8. Augenschutzmaske (1, 7) nach dem vorhergehenden Anspruch, **dadurch gekennzeichnet, dass** die Entnahmeeinrichtung eine Einführneigung (2224) enthält, die auf dem Clip (222, 223; 7322, 7323) eingerichtet und so angeordnet ist, dass die Einführneigung mit dem Knopf zusammenwirken kann, wenn der Knopf den Clip beansprucht, um eine Kraft auf den Clip in einer Richtung (X) zu erzeugen, die das Entfernen eines Teils des Schirms vom Rahmen im Bereich des Clips bewirkt.

9. Augenschutzmaske (1, 7) nach einem der Ansprüche 6 oder 7, **dadurch gekennzeichnet, dass** die Entnahmeeinrichtung eine Auswurfneigung (44) enthält, die auf dem Knopf (4, 74) eingerichtet und so angeordnet ist, dass die Auswurfneigung mit dem Clip zusammenwirken kann, wenn der Knopf den Clip beansprucht, um eine Kraft auf den Clip in einer Richtung (X) zu erzeugen, die das Entfernen eines Teils des Schirms vom Rahmen im Bereich des Clips bewirkt.

10. Augenschutzmaske (1; 7) nach einem der vorhergehenden Ansprüche, **dadurch gekennzeichnet, dass** eine Einhakeinrichtung (222, 223; 7322, 7323) vom Knopf verformbar ist, wenn dieser betätigt wird.

11. Augenschutzmaske (1) nach dem vorhergehenden Anspruch, **dadurch gekennzeichnet, dass** sie einen Anschlag (224a, 224b) enthält, der es ermöglicht, die Verformung einer Einhakeinrichtung (222, 223) zu begrenzen.

12. Augenschutzmaske (1; 7} nach einem der vorhergehenden Ansprüche, **dadurch gekennzeichnet, dass** der Kopf (41) des Knopfes in einem Hohlraum der Maske untergebracht ist.

13. Augenschutzmaske (1) nach einem der vorhergehenden Ansprüche, **dadurch gekennzeichnet, dass** der Knopf im Bereich einer seitlichen Ausdehnung (32) des Rahmens positioniert ist.

14. Augenschutzmaske (1) nach dem vorhergehenden Anspruch, **dadurch gekennzeichnet, dass** sie mindestens vier Knöpfe (4) enthält, die mit mindestens vier Clips (222a, 222b, 223a, 223b) zusammenwirken können, wobei jede seitliche Ausdehnung (32a, 32b) des Rahmens zwei entgegengesetzt montierte Knöpfe trägt.

15. Augenschutzmaske (7) nach einem der Ansprüche 1, 7 bis 10 oder 12, **dadurch gekennzeichnet, dass** der Knopf (74) im zentralen Teil des Schirms (72) untergebracht ist.

## Claims

1. Eye-protection mask (1; 7) for use in open-air sports, comprising:
- a frame (3; 73) delimiting an opening (31), the frame supporting at least one first latching means (322; 7322, 7323),
- an eye-protection screen (2; 72) that is intended to close off the opening, the screen supporting at least one second latching means (222, 223; 7222), the second latching means being able to cooperate directly with the first latching means so as to keep the screen secured to the frame in at least one direction (X),
- the mask is provided with a button (4; 74) that is able to push a latching means (222/223, 322; 7322/7323, 7222) such that it no longer cooperates with the other latching means (322, 222/223; 7222, 7322/7323),
**characterized in that** the button is mounted on the part (2, 3; 72, 73) which does not support the latching means that can be pushed by the button.

2. Eye-protection mask (1) according to Claim 1, **characterized in that** the latching means which is pushed by the button is the second latching means (222, 223) supported by the screen (2).

3. Eye-protection mask (1) according to the preceding claim, **characterized in that** the first latching means comprises a recess (322) and the second latching means comprises a clip (222, 223), a stop surface (2223), provided on the clip, being able to cooperate with a matching stop surface (3222), provided in the recess, the clip (222, 223) being pushed elastically so as to be able to return to a stable position in which the stop surface of the clip is able to cooperate with the matching stop surface of the recess.

4. Eye-protection mask (1) according to one of the preceding claims, **characterized in that** a part of the frame constitutes the first latching means.

5. Eye-protection mask (1) according to one of the preceding claims, **characterized in that** the frame comprises a housing (323) for guiding the button.

6. Eye-protection mask (1) according to the preceding claim, **characterized in that** the button (4) comprises at least one abutment surface (45, 46) that is able to cooperate with at least one matching abutment surface (3235, 3236) provided on the guide housing of the button so as to limit the movement of the button in one direction (Z).

7. Eye-protection mask (1, 7) according to one of the preceding claims, **characterized in that** the button and/or a latching means comprises an extraction means (44, 2224, 3223) that causes one part of the screen to move away from the frame when the button is actuated.

8. Eye-protection mask (1, 7) according to the preceding claim, **characterized in that** the extraction means comprises an insertion slope (2224) provided on the clip (222, 223; 7322, 7323) and arranged in such a way that the insertion slope can cooperate with the button, when the button pushes the clip, in order to produce a force on the clip in a direction (X) that causes a part of the screen to move away from the frame at the clip.

9. Eye-protection mask (1, 7) according to either of Claims 6 and 7, **characterized in that** the extraction means comprises an ejection slope (44) provided on the button (4, 74) and arranged in such a way that the ejection slope can cooperate with the clip, when the button pushes the clip, in order to produce a force on the clip in a direction (X) that causes a part of the screen to move away from the frame at the clip.

10. Eye-protection mask (1; 7) according to one of the preceding claims, **characterized in that** a latching means (222, 223; 7322, 7323) can be deformed by the button when the latter is actuated.

11. Eye-protection mask (1) according to the preceding claim, **characterized in that** it comprises an abutment (224a, 224b) that serves to limit the deformation of a latching means (222, 223).

12. Eye-protection mask (1; 7) according to one of the preceding claims, **characterized in that** the head (41) of the button is accommodated in a cavity of the mask.

13. Eye-protection mask (1) according to one of the preceding claims, **characterized in that** the button is positioned at a lateral extension (32) of the frame.

14. Eye-protection mask (1) according to the preceding claim, **characterized in that** it comprises at least four buttons (4) that are able to cooperate with at least four clips (222a, 222b, 223a, 223b), each lateral extension (32a, 32b) of the frame supporting two buttons mounted in opposition.

15. Eye-protection mask (7) according to one of Claims 1, 7 to 10 or 12, **characterized in that** the button (74) is accommodated in the central part of the screen (72).
